# EUROPEAN PATENT APPLICATION

(11) **EP 2 241 588 A1**
(43) Date of publication of application: **20.10.2010**
(21) Application number: 09707388.6
(22) Date of filing: 05.02.2009
(51) Int. Cl.: C08J 3/075, A61K 8/73, A61K 9/06, A61K 47/36, A61Q 19/00, C08J 3/28, C08L 3/00, C08L 5/00

(54) **AQUEOUS DISPERSION CONTAINING POLYSACCHARIDE PARTICULATE GEL AND METHOD FOR PRODUCING THE SAME**

(30) Priority: 06.02.2008 JP 2008026130
(71) Applicant: Toray Industries, Inc., Tokyo 103-8666 (JP)
(72) Inventor: OKUMURA, Hiroto, Otsu-shi Shiga 520-8558 (JP); SHIMAGAKI, Masaaki, Urayasu-shi Chiba 279-8555 (JP); TANIGUCHI, Takashi, Otsu-shi Shiga 520-8558 (JP)
(74) Representative: Kador & Partner
(86) International application number: PCT/JP2009/051930
(87) International publication number: WO 2009/099120

(57) **Abstract**

Disclosed is an aqueous dispersion containing polysaccharide particulate gels, which is **characterized by** containing particulate gels substantially composed of a polysaccharide. The aqueous dispersion containing polysaccharide particulate gels is also **characterized in that** shapes of the particulate gels are maintained even after the particulate gels are heated for 30 minutes at 95°C in an aqueous medium. Namely, disclosed is an aqueous dispersion containing polysaccharide particulate gels, wherein the particulate gels are composed of a polysaccharide and have shapes that can be maintained even after 30-minute heating in an aqueous medium of 95°C.

## Description

### TECHNICAL FIELD

The present invention relates to an aqueous dispersion containing polysaccharide particulate gel and a method for producing the same.

### BACKGROUND ART

In the fields of drugs, medical materials, foods, cosmetics, and so on, many products made of polysaccharides in various forms have been developed and gel-like products also have been proposed.

Compositions in which polysaccharides have been incorporated in order to improve easiness of application, living body safety, or drug retention have been known, and Patent Documents 1 to 3, for example, each disclose a polysaccharide-containing composition in which part or the whole of the polysaccharide is in the form of fine particles, which are dispersed uniformly in an aqueous medium and a method for producing the same by dissolving a polysaccharide in an aqueous medium at a temperature higher than the gel transition temperature and cooling the solution to or below the gel transition temperature while applying a shearing force.

Patent Document 4 discloses a polysaccharide-containing composition whose viscosity increases when it comes into contact with a physiological fluid containing a salt such as sweat and tear fluid, and the document describes that the composition can be applied as a base of a drug or cosmetic having a property that it can be applied or administered easily to a wide area because it has a low viscosity before use and it becomes difficult to stream down during use due to increase in viscosity during the use. In addition, regarding an ophthalmic application, it is also disclosed that the polysaccharide-containing composition has been found to have an effect of improving the transferability of a drug into the intraocular tissue, i.e., the ocular penetration and is useful as a base of an ophthalmic solution. Patent Document 5 discloses an effect of a polysaccharide-containing composition to remarkably stabilize the tear film of the eyeball surface and also discloses that the composition is useful as an ophthalmic solution for curing or preventing dry eye. Furthermore, Patent Document 6 discloses that a polysaccharide-containing composition exhibits a specific thickening action when it comes into contact with mucin contained in a tear film and also discloses that since it is excellent in drug retentivity in an anterior eye part and eliminates a dry feeling of an eye, it is useful as an eyeball surface retention promoter, a medicine for treating dry eye or an artificial tear.

However, the preparation of such a polysaccharide-containing composition is accompanied by risks such as (1) contamination of bacteria or microorganisms floating in the air or adhering to a reagent into the composition, and (2) contamination of bacteria into the composition when the composition is exposed to the air or is brought into contact with a skin or the like during the preservation of the composition. In order to eliminate such risks and obtain a polysaccharide-containing composition that is high in living body safety, it is necessary to perform some sterilization operations to kill bacteria or microorganisms that have entered the polysaccharide-containing composition. Examples of such sterilization operations include filtration sterilization and heat sterilization. However, filtration sterilization is a method of removing bacteria or microorganisms from a composition by using a filter having countless holes of a fixed size and cannot remove minute microorganisms smaller than the hole diameter of the filter. Moreover, in the case where the particulate material made of a polysaccharide is larger than the hole diameter of a filter, filtration sterilization is not appropriate as a sterilization method of the polysaccharide-containing composition because the particulate material is removed together with bacteria or microorganisms.

On the other hand, such methods as flame sterilization, dry heat sterilization, or autoclaved sterilization (high pressure steam sterilization) can be mentioned as heat sterilization. These sterilization methods are methods of killing bacteria or microorganisms that have entered a composition by heating the composition at a high temperature. Autoclaved sterilization is preferable as a sterilization method of a composition containing a particulate material made of a polysaccharide. However, when the composition containing the particulate material made of the polysaccharide is an aqueous dispersion, there is a problem that autoclaved sterilization is difficult to be applied to drugs, medical materials, foods or cosmetics because if heating is carried out at a temperature equal to or higher than the gel transition temperature of the polysaccharide, more specifically, a temperature of 90°C or higher, the particulate material made of the polysaccharide dissolves and therefore the resultant must be cooled to the gel transition temperature or lower while applying a shearing force and, as a result, the properties of the polysaccharide-containing composition before and after heating are different. Therefore, the development of a polysaccharide-containing composition in which the form of a particulate material made of the polysaccharide is maintained even if heating at a temperature equal to or higher than the gel transition temperature of the polysaccharide is carried out has been expected.

Furthermore, in the fields of drugs or cosmetics, the development of a product that has high moisture retentivity and is excellent in living body safety has been expected, and polyhydric alcohols, natural polymers or polysaccharides have been used mainly as effective components of humectants for inhibiting desiccation of the skin and holding moisture of the skin. Particularly, from the viewpoint of securing high moisture retentivity and high living body safety, the development of a humectant which contains a polysaccharide as an active component has been carried out actively. For example, Patent Document 7 discloses a humectant which contains hyaluronic acid having a molecular weight of 2000000 or more and exerts improved comfort in use. Patent Document 8 discloses a humectant that contains a polysaccharide as an active component and has a stable moisture retentivity. However, although it is preferable to incorporate a polysaccharide in a high concentration to a humectant in order to secure sufficient moisture retentiveity and sufficient living body safety, there is a problem that the high concentration incorporation of a polysaccharide is accompanied by the occurrence of stickiness so that the comfort in use lowers. Therefore, the amount of the polysaccharide contained in the humectant is about 0.1 to about 0.3% by weight at most relative to the whole portion of the humectant. Therefore, the development of a humectant which does not generate stickiness despite incorporation of a polysaccharide in a high concentration and which is excellent in moisture retentivity, comfort in use and living body safety has been expected.

As a therapeutic method for eye diseases such as glaucoma and cataract, a method of applying a drug directly to an eye, i.e., drug instillation, is common. Although the therapeutic effect on eye diseases exerted by the drug instillation depends, of course, on the effect of the drug itself, in order to make the drug exert its effect sufficiently, to make the effect of the drug instilled be maintained for a long time, that is, to increase the drug retentivity becomes an important task.

Moreover, frequent occurrence of dry eye symptoms has been regarded as a problem because a long-time work using a computer or the use of a contact lens has recently been routinized. The tear film covering the eyeball surface is very thin and is kept smooth. However, if the tear film becomes unstable, the surface thereof becomes unsmooth so that a dry part called a dry spot will be formed in a short time before winking and part of cornea may be exposed. This causes unusual desiccation of an eye part or a defect of a tear film (i.e., dry spot) and, as a result, it is highly possible to cause a serious disorder in an external eye part such as a cornea and a conjunctiva. In order to prevent or cure such a dry eye symptom, it is required to stabilize the tear film on the eyeball surface for a long time and keep the tear film on the eyeball surface smooth.

Various researches for making the efficacy of a drug last effectively have been made. For example, Patent Document 9 discloses a technology of improving the ocular drug penetration by increasing the drug retention on the eyeball surface and the controlled releasability of a drug by increasing the viscosity of an ophthalmic solution itself by using a carboxyvinyl polymer (CVP) as a base. The technology uses a characteristic of CVP, i.e., a property to significantly increase the viscosity of a drug even in a very small added amount.

However, although the technology can be used preferably when the dosage form is an ophthalmic ointment, it is unsuitable for application to an ophthalmic solution which needs to be applied in a state of droplet. Therefore, the development of an ophthalmic solution which can be applied in the form of droplet and can attain a high ocular drug penetration has been expected.

Patent Document 10 discloses an ophthalmic composition containing a poly-γ-glutamic acid (PGA) as an effective component and describes that a composition for a ophthalmic solution which is useful for preventing and curing dry eye or a composition for an artificial tear which maintains an effect as a tear substitute for a long time can be obtained. Although it has been recommended that the molecular weight of the PGA to be used as a base of the ophthalmic composition be 50000 or more in order to increase the viscosity of the ophthalmic composition and improve the property to include a water molecule, when using such a high molecular weight body as a base, it becomes difficult to control properties of the composition for an ophthalmic solution or the composition for an artificial tear relating to the ease in handling or the drug effect as a product (e.g., viscosity or solubility in a solvent) so that they may fall within certain ranges. For example, although the ophthalmic composition is required to have a low viscosity, if a high molecular weight body is used as a base, the viscosity easily varies when preparing a low viscosity composition and it becomes difficult to produce such a composition.

On the other hand, it has been known that the compositions containing a particulate material made of a polysaccharide disclosed in Patent Documents 1 to 6 are excellent in ocular drug penetration, further have an action to remarkably stabilize a tear film on the eyeball surface and can be used as a base of an ophthalmic composition such as an ophthalmic solution or a preservative solution for preventing or curing dry eye. Moreover, it also has been known that when using the compositions as a base of an ophthalmic composition, it is possible to produce a high quality product with small variation in effects relating to ease in handling or drug effect.

However, generally in order to market these ophthalmic compositions as products, it is necessary to completely remove bacteria, microorganisms, and the like contained in the ophthalmic compositions by performing a sterilization operation represented by autoclaved sterilization for securing living body safety. However, if autoclaved sterilization is performed to an ophthalmic composition containing a polysaccharide-containing composition as a base, a particulate material made of the polysaccharides will dissolve. Moreover, there also has been a problem that the ophthalmic composition containing a polysaccharide-containing composition as a base cannot be applied to practical products such as an ophthalmic solution and a preservative solution for contact lenses because the viscosity of the ophthalmic composition increases by at least several tens mPa·s in comparison to that before the sterilization operation. Therefore, the development of an ophthalmic composition containing, as a base, a polysaccharide-containing composition that can be heat sterilized and can maintain its properties before heat sterilization also after performing the sterilization has been expected.

Patent Document 1: JP 2-191540 A
Patent Document 2: JP 2004-244329 A
Patent Document 3: JP 2005-162955 A
Patent Document 4: JP 2003-128588 A
Patent Document 5: JP 2005-163023 A
Patent Document 6: JP 2006-89460 A
Patent Document 7: JP 63-156707 A
Patent Document 8: JP 8-301904 A
Patent Document 9: JP 60-56684 B
Patent Document 10: JP 2006-327949 A

### DISCLOSURE OF THE INVENTION

### PROBLEM TO BE SOLVED BY THE INVENTION

In light of such situations, an object of the present invention is to provide an aqueous dispersion containing a polysaccharide particulate gel that is a composition in which a particulate material made of a polysaccharide maintains its particle form even if it is heated, in other words, a polysaccharide particulate gel maintains the form of the polysaccharide particulate gel without being dissolved even if it is heated and the polysaccharide particulate gel is dispersed uniformly in an aqueous medium, and a method for producing the same, for the purpose of applying to drugs, medical materials, foods, cosmetics, and so on.

### MEANS FOR SOLVING THE PROBLEM

The present inventors studied earnestly in order to solve such problems and, as a result, have accomplished the present invention by finding out a polysaccharide particulate gel the form of which is maintained also after being heated in an aqueous medium.

That is, the present invention is as stated in the following (1) to (10).
(1) The present invention provides an aqueous dispersion containing a polysaccharide particulate gel **characterized in that** the aqueous dispersion contains a particulate gel composed of substantially a polysaccharide and the shape of the particulate gel is maintained after the particulate gel has been heated for 30 minutes in an aqueous medium of 95°C, in other words, an aqueous dispersion containing a polysaccharide particulate gel, wherein shape of the particulate gel is maintained after the particulate gel has been heated for 30 minutes in an aqueous medium of 95°C.
(2) The present invention provides the aqueous dispersion containing a polysaccharide particulate gel described in (1) above which is **characterized in that** the particulate gel of the preceding item is obtained by irradiating the polysaccharide in an aqueous medium with an ionizing radiation, in other words, the aqueous dispersion containing a polysaccharide particulate gel described in (1) above, wherein the particulate gel is one that has been irradiated with ionizing radiation in an aqueous medium.
(3) The present invention provides the aqueous dispersion containing a polysaccharide particulate gel described in (2) above which is **characterized in that** the polysaccharide in the aqueous medium of the preceding item is in the form of fine particles, in other words, the aqueous dispersion containing a polysaccharide particulate gel described in (1) or (2) above, wherein the particulate gel is 500 µm or smaller in particle diameter.
(4) The present invention provides the aqueous dispersion containing a polysaccharide particulate gel described in (2) or (3) above which is **characterized in that** the ionizing radiation is γ rays or electron rays, in other words, the aqueous dispersion containing a polysaccharide particulate gel described in (2) or (3) above, wherein the ionizing radiation is γ rays or electron rays.
(5) The present invention provides a method for producing an aqueous dispersion containing a polysaccharide particulate gel, the method being characterized by being composed of a polysaccharide and an aqueous medium and comprising a step of heating to or above the gel transition temperature of the polysaccharide, thereby dissolving the polysaccharide in the aqueous medium, a step of cooling the polysaccharide dissolved in the aqueous medium to or below the gel transition temperature while applying an external force, thereby obtaining particulate polysaccharide, and a step of irradiating the aqueous dispersion containing the particulate polysaccharide with ionizing radiation, in other words, a method for producing the aqueous dispersion containing a polysaccharide particulate gel according to any one of (1) to (4) above, the method comprising: a dissolving step of heating a polysaccharide in an aqueous medium to or above a gel transition temperature, thereby dissolving the polysaccharide in the aqueous medium, a cooling step of cooling the polysaccharide dissolved in the aqueous medium to or below the gel transition temperature while applying an external force, thereby making the polysaccharide into a particulate gel, and an irradiation step of irradiating the particulate gel in the aqueous medium with ionizing radiation, thereby obtaining an aqueous dispersion containing a polysaccharide particulate gel.
(6) The present invention provides the method for producing the aqueous dispersion containing a polysaccharide particulate gel described in (5) above **characterized in that** the ionizing radiation is γ rays or electron rays, in other words, the production method described in (5) above, wherein the ionizing radiation is γ rays or electron rays.
(7) The present invention provides a humectant containing a polysaccharide particulate gel using the aqueous dispersion containing a polysaccharide particulate gel described in (1) above, wherein the humectant comprises a particulate gel composed of substantially a polysaccharide in an amount of 0.01 to 5% by weight, and the average particle diameter of the particulate gel is 1 µm to 200 µm, in other words, a humectant containing a polysaccharide particulate gel, wherein the humectant contains the aqueous dispersion containing a polysaccharide particulate gel described in (1) to (5) above, the content of the particulate gel is 0.01 to 5% by weight, and the average particle diameter of the particulate gel is 1 to 200 µm.
(8) The present invention provides the humectant containing a polysaccharide particulate gel described in (7) above, wherein at least one additive selected from among glycerine and an alkyl parahydroxybenzoate (the alkyl group contains one or two carbon atoms), in other words, the humectant containing a polysaccharide particulate gel described in (7) above, wherein glycerine and/or an alkyl parahydroxybenzoate having an alkyl group having one or two carbon atoms has been added thereto.
(9) The present invention provides an ophthalmic composition containing a polysaccharide particulate gel using the aqueous dispersion containing a polysaccharide particulate gel described in (1) above, wherein the composition comprises a particulate gel composed of substantially a polysaccharide in an amount of 0.0001 to 1% by weight, and the average particle diameter of the particulate gel is 0.1 µm to 100 µm, in other words, an ophthalmic composition containing a polysaccharide particulate gel, wherein the composition contains the aqueous dispersion containing a polysaccharide particulate gel described in any one of (1) to (5) above, the content of the particulate gel is 0.0001 to 1% by weight, and the average particle diameter of the particulate gel is 0.1 to 100 µm.
(10) The present invention provides an ophthalmic solution containing the ophthalmic composition containing a polysaccharide particulate gel described in (9) above, in other words, an ophthalmic solution comprising the ophthalmic composition containing a polysaccharide particulate gel described in (9) above.

### EFFECT OF THE INVENTION

The aqueous dispersion containing a polysaccharide particulate gel of the present invention has new functions and can provide pharmaceuticals, medical materials, foods and cosmetics high in living body safety because the polysaccharide particulate gel does not dissolve even if heat sterilization is performed at or above the gel transition temperature of the polysaccharide and the form of the polysaccharide particulate gel is maintained after the heating. More specifically, it can provide, for example, sun-block cream that is high in living body safety and is hardly washed away by sea water, cosmetics or medical ointment which is not washed away by sweat, ophthalmic solutions whose medicinal ingredients is inhibited from being flowed away by tear, artificial tears which stabilize the tear film located on the surface of an eyeball, preparations for nasal cavities and oral cavities which stay in a nasal cavity and an oral cavity for a long time and are good in durability of efficacy. Moreover, by using a property that the viscosity increases when the aqueous dispersion comes into contact with mucin located on a mucous membrane surface of a trachea or a sexual gland, it becomes possible to apply the aqueous dispersion to a drug delivery system which stays at an affected part for a long time and efficiently releases a drug slowly. BRIEF DESCRIPTION OF THE DRAWINGS

[Fig. 1] An optical microscope photograph of the aqueous dispersion B obtained in Example 1, taken before heating.
[Fig. 2] An optical microscope photograph of the aqueous dispersion B obtained in Example 1, taken after heating.
[Fig. 3] An optical microscope photograph of the aqueous dispersion C obtained in Example 2, taken before heating.
[Fig. 4] An optical microscope photograph of the aqueous dispersion C obtained in Example 2, taken after heating.
[Fig. 5] An optical microscope photograph of the aqueous dispersion D obtained in Example 3, taken before heating.
[Fig. 6] An optical microscope photograph of the aqueous dispersion D obtained in Example 3, taken after heating.
[Fig. 7] An optical microscope photograph of the aqueous dispersion B obtained in Example 4, taken before heating.
[Fig. 8] An optical microscope photograph of the aqueous dispersion B obtained in Example 4, taken after heating.
[Fig. 9] An optical microscope photograph of the aqueous dispersion A obtained in Comparative Example 1, taken before heating.
[Fig. 10] An optical microscope photograph of the aqueous dispersion A obtained in Comparative Example 1, taken after heating.

### BEST MODE FOR CARRYING OUT THE INVENTION

The aqueous dispersion containing a polysaccharide particulate gel of the present invention is **characterized in that** it contains a particulate gel composed of a polysaccharide and that the shape of the particulate gel is maintained after the particulate gel has been heated for 30 minutes in an aqueous medium of 95°C.

The "particulate gel composed of a polysaccharide" means a particulate gel about 90 to 100% of the constituents of which is accounted for by a polysaccharide. Although generally, a particulate gel composed of a polysaccharide is dissolved when being heated in an aqueous medium of 90°C or higher, the polysaccharide particulate gel according to the present invention does not dissolve and the particulate form is maintained even after the gel has been heated in an aqueous medium of 95°C.

The "aqueous medium" refers to a liquid substance containing water as a main component and components other than water are not restricted particularly. One having a content of water of more than 70 to 80 wt% is preferable and one having a content of water of more than 90 wt% is more preferable.

The aqueous medium may contain a water-soluble compound which dissolves in water to give a stable composition.

Examples of the water-soluble compound include alcohols such as methanol, ethanol, ethylene glycol, propylene glycol and glycerine, or water-soluble low molecular weight compounds such as a surfactant, an emulsifier, a dispersing agent, and a tonicity adjusting agent, or water-soluble polymer compounds such as polyethylene glycol or polyvinyl alcohol. Each of these may be contained alone, or alternatively two or more of these may be contained in combination.

The tonicity adjusting agent refers to a solute contained in an isotonic solution. The isotonic solution referred to herein is a solution which is originally one of two or more solutions differing in osmotic pressure and into which an appropriate amount of tonicity adjusting agent has been added so that the osmotic pressure may become the same. When the composition in the present invention, that is, the aqueous dispersion containing a polysaccharide particulate gel is used singly or in combination with multiple compositions, a tonicity adjusting agent may be used.

Examples of the tonicity adjusting agent include sorbitol, mannitol, sodium chloride, sodium phosphate, boric acid, or glycerine.

Although the polysaccharide particulate gel according to the present invention is **characterized in that** the form thereof is maintained even after being heated in an aqueous medium of 95°C for 30 minutes, autoclaved sterilization also can be used because the gel does not dissolve even after being heated in an aqueous medium of 121°C for 20 minutes in order to completely kill the microorganisms present in an aqueous dispersion containing the polysaccharide particulate gel by using autoclaved sterilization.

In order to obtain the polysaccharide particulate gel according to the present invention which is **characterized in that** the form thereof is maintained also after being heated, it is preferable to irradiate a polysaccharide particulate gel present in an aqueous medium with ionizing radiation. The ionizing radiation is preferably applied to a polysaccharide particulate gel located in an aqueous medium because if the ionizing radiation is applied directly to a polysaccharide particulate gel, a decomposition reaction is promoted so that a polysaccharide particulate gel that does not dissolve even after being heated cannot be obtained. It is preferable that a polysaccharide gel located in an aqueous medium have a particulate form because a reaction efficiency achieved by the application of ionizing radiation is high.

Generally, radiation is divided roughly into ionizing radiation and non-ionizing radiation, and "ionizing radiation" refers to radiation that has an action of ejecting electrons from atoms that form a substance through which the radiation penetrates, the atoms being located in the path of the radiation. Examples of the ionizing radiation include γ rays, electron rays, X rays, β rays, or α rays. On the other hand, examples of the non-ionizing radiation include ultraviolet rays or visible rays. Since non-ionizing radiation is very weak in the action of ejecting electrons from atoms, it is preferable, from the viewpoint of a reaction efficiency by irradiation, to apply ionizing radiation, more preferably to apply γ rays or electron rays. It is even more preferable, from the economical point of view, to apply electron rays.

Although the mechanism of the development of the feature that the form of the polysaccharide particulate gel according to the present invention is maintained also after heating has not been elucidated completely, it is presumed that if a polysaccharide particulate gel in an aqueous medium is irradiated with ionizing radiation, a water molecule itself generates a hydroxyl radical and this hydroxyl radical induces the start of a crosslinking reaction of a molecular chain constituting the polysaccharide particulate gel. Moreover, when part of a molecular chain constituting the polysaccharide particulate gel have comparatively free molecular mobility, it is presumed that the feature is developed because a crosslinking site induced by the hydroxyl radical generated by the ionizing radiation can approach to a distance short enough for causing a reaction with another molecular chain.

The polysaccharide that constitutes the polysaccharide particulate gel according to the present invention is not particularly restricted, and it may be any of ordinary polysaccharides such as those described in "Basics of Glycochemistry" written by Kimiko Abu and Nobuko Seno, published by Kodansha in 1984. Although a plurality of polysaccharides may be used together, polysaccharides obtained from plants are preferable. Examples of the polysaccharide obtained from plants include agar, agarose, agaropectin, starch, amylose, amylopectin, isolichenan, laminaran, lichenan, glucan, inulin, levan, fructan, galactan, mannan, xylan, arabinan, pentozan, alginic acid, pectinic acid, fucoidan, ascophyllan, carrageenan, pectin, locust bean gum, guar gum, tara gum, or arabic gum. Polysaccharides obtained from seaweed such as agar, agarose, agaropectin, laminaran, fructan, galactan, pentozan, alginic acid, chitin, porphyran, fucoidan, ascophyllan, and carrageenan are preferable, agar, agarose, and agaropectin are more preferable, and agar is even more preferable.

Agar has already been used widely for foods and the like and can be said to be highly safe to living bodies because it is listed in Japanese Pharmacopoeia. It has been known that agar is useful as a component that increase the moisture retentivity of drugs, medical materials, foods, cosmetics, and so on because agar has a moisture retentive action of inhibiting moisture from evaporating.

Agar is a kind of polysaccharide contained in the cell wall matrix of various red algae such as Gelidiales and Gracilariaceae, which is obtained from red alga by extraction using hot water. Agar is not a uniform substance and typically consists of agarose, which contain no sulfuric acid groups, and agaropectin, which contains a sulfuric acid group and the like. The proportion of agarose varies depending on the type of the red alga, and agarose accounts for about 70% in Gelidiales, for example.

Although the agar to be used for the execution of the present invention may be a product produced by any production process, an agar produced by an industrial production process is preferable from the viewpoint of stable supply, and the weight average molecular weight thereof is preferably 5000 to 1200000, more preferably 30000 to 800000, and even more preferably 50000 to 500000.

Although the shape of the polysaccharide particulate gel according to the present invention may be, for example, a spherical shape, and elliptical shape, or an indefinite shape, it is preferably a spherical shape from the viewpoint of reducing feeling of foreign substance or unpleasantness.

A coarse polysaccharide particulate gel having a particle diameter of several hundreds µm have an adverse effect on the storage stability of a composition, that is, an aqueous dispersion containing a polysaccharide particulate gel and it may cause inconvenience in functions such as unpleasantness or stimulus on an eyeball or in a nasal cavity, when it is used as an ophthalmic solution or a preparation for a nasal cavity or an oral cavity. Therefore, the particle diameter of the polysaccharide particulate gel is preferably 500 µm or less, more preferably 100 µm, and even more preferably 30 µm or less.

The aqueous dispersion containing a polysaccharide particulate gel of the present invention can be applied also as a base of a medicine or a medical material. As a medicinal ingredient contained in such a base, i.e., a drug, drugs that are practically used clinically or drugs which are expected to be used clinically can be mentioned widely.

Examples of the drugs include hypnotic-sedative agents such as glutethimide, chloral hydrate, nitrazepam, amobarbital, and phenobarbital, analgesic-antipyretic-antiphlogistic drugs such as aspirin, acetaminophen, ibuprofen, flurbiprofen, indomethacin, Ketoprofen, diclofenac sodium, tiaramide hydrochloride, piroxicam, flufenamic acid, mefenamic acid, and pentazocine, local anesthetics such as methyl aminobenzoate and lidocaine, local vasoconstrictors such as naphazoline nitrate, tetryzoline nitrate, oxymethazone hydrochloride, and tramazoline hydrochloride, antiallergic agents such as chlorpheniramine maleate, sodium cromoglicate, oxatomide, azelastine hydrochloride, ketotifen fumarate, traxanox sodium, and amlexanox, germicides such as benzethonium chloride, cardiotonics such as dopamine hydrochloride and ubidecarenone, antiarrhythmic agents such as propranolol hydrochloride, pindolol, phenytoin, and disopyramide, coronary vasodilators such as isosorbide dinitrate, nifedipine, diltiazem hydrochloride, and dipyridamole, agents for digestive organs such as domperidone, adrenal corticosteroids such as triamcinolone acetonide, dexamethasone, betamethasone sodium phosphate, prednisolone acetate, fluocinonide, beclometasone dipropionate, and flunisolide, antiplasminic agents such as tranexamic acid, antifungals such as clotrimazole, miconazole nitrate, and ketoconazole, anti-malignant tumor agents such as tegafur, fluorouracil, and mercaptopurine, antibiotics such as amoxicillin, ampicillin, cephalexin, cephalotin sodium, ceftizoxime sodium, erythromycin, and oxytetracycline hydrochloride, calcitonins such as an insulin, calcitonin salmon, calcitonin chicken, and elcatonin, biologically active peptide such as urokinase, TPA, and interferon, vaccines such as influenza vaccine, pig Bordetella infection prophylactic vaccine, and hepatitis B vaccine, drugs for parasitic skin disease such as bifonazole, siccanin, bisdecalinium acetate, clotrimazole, and salicylic acid, drugs for suppurative disease such as sulfamethoxazole sodium, erythromycin, and gentamicin sulfate, antipruritics such as diphenhydramine, antimicrobials for external use such as iodine, povidone iodine, benzalkonium chloride, and chlorhexidine gluconate, antihistamines such as diphenhydramine hydrochloride and chlorpheniramine maleate, agents for sexual organs such as clotrimazole, naphazolyl nitrate, ketotifen fumarate, and miconazole nitrate, agents for otolaryngology such as tetryzoline hydrochloride, bronchodilators such as aminophylline, antimetabolites such as fluorouracin, hypnotic-sedative agents such as diazepam, and synthetic antibacterial agents such as norfloxacin and nalidixic acid.

The incorporation amount of the drug varies depending upon the kind of the drug, and the drug may generally be incorporated in an amount large enough for developing a desired effect of the drug.

To the aqueous dispersion containing a polysaccharide particulate gel of the present invention may be blended, as needed, medically acceptable agents such as a buffer, a pH regulator, a solubilizing agent, a stabilizer, or a preservative agent, when the aqueous dispersion is used for medical applications.

Examples of the buffer include phosphoric acid, citric acid, acetic acid, ε-aminocaproic acid, boric acid, borax, or trometamol. Such a buffer is preferably added in an amount necessary for maintaining the pH of the composition from 3 to 10.

Examples of the pH regulator include hydrochloric acid, citric acid, phosphoric acid, acetic acid, sodium hydroxide, potassium hydroxide, boric acid, borax, sodium carbonate, or sodium hydrogencarbonate.

Examples of the stabilizer include edetic acid or sodium edetate.

Examples of the preservative include sorbic acid, potassium sorbate, benzalkonium chloride, benzethonium chloride, methylparabene, propylparabene, or chlorobutanol, and these may be added in combination.

Examples of the solubilizing agent, which is added when the drug or the additives such as the buffer is slightly soluble in water, include polysorbate 80, polyoxyethylene hydrogenated castor oil, macrogol 4000, or cyclodextrin. It is preferably added in the range of 0.001 to 15% by weight.

The method for producing the aqueous dispersion containing polysaccharide particulate gel of the present invention is characterized by having a dissolving step of heating a polysaccharide in an aqueous medium to or above a gel transition temperature, thereby dissolving the polysaccharide in the aqueous medium, a cooling step of cooling the polysaccharide dissolved in the aqueous medium to or below the gel transition temperature while applying an external force, thereby making the polysaccharide into a particulate gel, and an irradiation step of irradiating the particulate gel in the aqueous medium with ionizing radiation, thereby obtaining an aqueous dispersion containing a polysaccharide particulate gel.

The "dissolving step of heating a polysaccharide in an aqueous medium to a gel transition temperature or higher, thereby dissolving the polysaccharide in the aqueous medium" can be performed according to the methods disclosed in known examples. For example, it can be performed by heating a composition containing a polysaccharide having a gel transition temperature and an aqueous medium at a temperature that is equal to or higher than the gel transition temperature of the polysaccharide while applying an external force to the composition, thereby dissolving the polysaccharide in the aqueous medium as disclosed in Patent Documents 1 to 4.

The "gel transition temperature" is a temperature at which the polysaccharide dissolved by heating becomes gel by being cooled and is a physical property value inherent to the polysaccharide. Therefore, a temperature equal to or higher than the gel transition temperature that is used in dissolving the polysaccharide in the aqueous medium, that is, a heating temperature is determined according to the kind of the polysaccharide to be used and is preferably a temperature that is equal to or higher than (the gel transition temperature + 20°C).

The amount of the polysaccharide to be dissolved in the aqueous medium is preferably within the range of 0.0001 to 30% by weight because if the amount of the polysaccharide to be dissolved in the aqueous medium is excessively small, the gelation effect exhibited by the polysaccharide will not be exerted sufficiently and if the content is excessively large, a composition with a low viscosity cannot be obtained.

During the dissolving step, an external force may be applied to the aqueous medium. Although examples of the means for applying the external force include vibration, shearing, stirring, compression, or pulverization, stirring is preferred because the external force is applied to a liquid.

Although examples of the stirring device for applying an external force to a liquid include stirring instruments such as a magnetic stirrer, a mechanical stirrer, a mixer, a shaker, or a rotor, a mechanical stirrer is preferred because no heat is generated with stirring and it can apply a large external force to a liquid uniformly. The rotation speed of the mechanical stirrer is preferably 1000 rpm or less.

The "cooling step of cooling the polysaccharide dissolved in the aqueous medium to the gel transition temperature or lower while applying an external force, thereby making the polysaccharide into a particulate gel" can be performed according to the methods disclosed in known examples. For example, like the method disclosed in Patent Document 3, a polysaccharide-containing aqueous dispersion in which part or the whole of the polysaccharide is in the form of fine particles and the particles are dispersed uniformly in the aqueous medium can be obtained by cooling the polysaccharide dissolved in the aqueous medium to or below the gel transition temperature at a cooling rate within a prescribed deviation while applying an external force. A temperature equal to or lower than the gel transition temperature that is used in cooling the polysaccharide dissolved in the aqueous medium, that is, a cooling temperature is preferably near room temperature, and more specifically, it is preferably within the range of 15 to 30°C because when it is equal to or lower than the gel transition temperature, gelation takes place microscopically partly, but if it is excessively high, part of the polysaccharide gelates ununiformly in the course of temperature lowering from that temperature to near room temperature and if it is excessively low, the molecular movement of the polysaccharide will be restrained so that the viscosity of the composition will increase.

Although examples of the means for applying an external force to the aqueous medium in which the polysaccharide has been dissolved include stirring instruments such as a magnetic stirrer, a mechanical stirrer, a mixer, a shaker, or a rotor, a mechanical stirrer is preferred because no heat is generated with stirring and it can apply a large external force to a liquid uniformly. Gelation advances with cooling and the viscosity of the polysaccharide-containing aqueous medium increases as the temperature lowers. However, if no external force is applied, the whole aqueous medium is gelated and even if an external force is applied after the whole aqueous medium has been gelated, an aqueous dispersion in which polysaccharide fine particles are dispersed uniformly cannot be obtained. It is preferable to continue stirring also after the temperature of the aqueous medium in which the polysaccharide has been dissolved has reached the gel transition temperature, and it is more preferable to continue stirring also after the even after reaching a temperature 20°C or more lower than the gel transition temperature, and it is more preferable to continue stirring also after reaching room temperature (30°C or lower).

Although examples of means for cooling the aqueous medium in which the polysaccharide has been dissolved include air cooling, water cooling, ice cooling, solvent cooling, or air blast cooling and air cooling or water cooling is general, it may be chosen appropriately depending upon the properties of the polysaccharide dissolved or the composition to be obtained, that is, the aqueous dispersion containing a polysaccharide particulate gel.

By the "irradiation step of irradiating the particulate gel in the aqueous medium with ionizing radiation, thereby obtaining an aqueous dispersion containing a polysaccharide particulate gel", a property that a polysaccharide particulate gel does not dissolve even if it is heated at high temperatures and the form of the polysaccharide particulate gel after heating is maintained can be imparted to a polysaccharide particulate gel. As the method for imparting such a property to a polysaccharide particulate gel, chemical methods in which a crosslinking agent or a reactive resin is added can be mentioned in addition to the method of applying ionizing radiation.

For example, JP 8-27277 A discloses a method for producing water-insoluble polysaccharide fine particles by producing fine particles by spray drying a solution in which a water-soluble polysaccharide and a reactive resin have been dissolved, and heat treating the resulting fine particles to chemically crosslink them. Moreover, JP 2005-82527 A discloses a production method which comprises heat dissolving a polysaccharide in an aqueous medium, then adding a crosslinking agent during a step of performing cooling while applying an external force, and thereby chemically crosslinking the polysaccharide. However, such chemical methods include addition of an initiator or a crosslinking agent and, therefore, may cause problems that are undesirable from a viewpoint of living body safety. For this reason, irradiation of ionizing radiation, which does not require addition of an initiator or a crosslinking agent, is preferable.

Examples of the ionizing radiation with which a polysaccharide particulate gel in an aqueous medium is irradiated include γ rays, electron rays, X rays, β rays, or α rays.

Since γ rays are greater in penetrating power than electron rays, γ rays are ray sources suitable for irradiation to such a sample that the distance from the sample liquid surface to the bottom of a container containing the sample is long. There is an advantage that the kind of the container into which the sample is put is not restricted as long as γ rays can penetrate. For example, it is also possible to perform irradiation treatment while putting a large amount of sample in a large drum or a stainless steel container. The γ-ray irradiation includes irradiation using cesium 137 and irradiation using cobalt 60. Since cesium 137 is inferior in irradiation efficiency to cobalt 60, γ-ray irradiation using cobalt 60 is preferable.

Unlike γ rays, electron rays does not use radioactive isotopes such as cobalt 60 or cesium 137. Moreover, a shielding apparatus may be comparatively simple one and electron rays have a feature of having a greater irradiation treatment ability in comparison to γ rays. Therefore, electron rays are ray sources preferable in a safety aspect or a working aspect.

The factors which control the irradiation of γ rays or electron rays are a dose for γ rays and a dose and an exposure voltage for electron rays. The dose means that the energy absorption is 1 J for 1 kg of a substance to be irradiated. The unit thereof is expressed by Gy (gray). The irradiation voltage is correlated with the depth of penetration into an aqueous dispersion of a polysaccharide particulate gel to be irradiated and it is meant that if the voltage is increased, the depth of penetration increases. The dose required for a crosslinking reaction is preferably a dose of about 1 to 300 kGy and more preferably a dose of 2 to 50 kGy because if the dose is less than 1 kGy, the crosslinking reaction proceeds insufficiently and if the dose exceeds 300 kGy, a decomposition reaction proceeds.

Electron rays, the penetrating power of which (i.e., energy of electron rays) changes with the exposure voltage, are generally lower in penetrating power in comparison to γ rays. For this reason, it is preferable to adjust the liquid depth of a sample according to the penetrating power of the electron rays to be used. For example, electron rays having a high energy of about 10 MeV can uniformly irradiate a sample having a liquid depth of several millimeters to about 3 cm, whereas electron rays having a lower energy of 1 MeV or less can uniformly irradiate only samples having a liquid depth of up to about 3 mm.

The humectant containing a polysaccharide particulate gel of the present invention is **characterized in that** the humectant contains the aforementioned aqueous dispersion containing a polysaccharide particulate gel, the content of the particulate gel is 0.01 to 5% by weight, and the average particle diameter of the particulate gel is 1 to 200 µm.

The polysaccharide particulate gel according to the present invention can be used as a constituent of a humectant having a pharmaceutical form such as ointment, cream, or lotion. The polysaccharide particulate gel according to the present invention can be heat-sterilized and, moreover, it is excellent in comfort in use because it can inhibit occurrence of stickiness even if it is blended at a high concentration. Furthermore, the moisture retaining ability of the whole humectant can be greatly increased by blending at a high concentration because the polysaccharide particulate gel itself is high in moisture retaining ability. The content of the polysaccharide particulate gel is preferably 0.001 to 10% by weight and more preferably 0.01 to 5% by weight because if the blended amount relative to the whole humectant is less than 0.001% by weight, the effect thereof will not be demonstrated sufficiently and if the blended amount exceeds 10% by weight, the comfort in use of the humectant will deteriorate.

If the average particle diameter of the polysaccharide particulate gel is larger than 500 µm, adverse effects will be inflicted on storage stability of a humectant and the comfort in use to be felt on application to the skin will be deteriorated. Therefore, the average particle diameter is preferably 0.1 to 500 µm, more preferably 1 to 300 µm, and even more preferably 1 to 200 µm.

Antiseptics or other moisturizing components, for example, may be blended to the humectant containing the polysaccharide particulate gel of the present invention according to the kind or the purpose of the humectant.

Examples of the antiseptics include sorbic acid, benzalkonium chloride or alkyl parahydroxybenzoate.

Although examples of other humectants include chlorobutanol, sodium chloride, magnesium chloride, sodium sulfate, glycerine, propylene glycol, xylitol, proline, serine, or glycine, glycerine, propylene glycol, methyl parahydroxybenzoate, or ethyl p-hydroxybenzoate is preferable from the viewpoints of safety, stability, and economical efficiency. Moreover, glycerine or methyl parahydroxybenzoate is more preferable because these have good affinity with polysaccharides and are excellent in dispersibility caused by being made present together with an alcohol such as methanol or ethanol, and can demonstrate an effect as an additive sufficiently.

Furthermore, the ophthalmic composition containing a polysaccharide particulate gel using the aqueous dispersion containing a polysaccharide particulate gel of the present invention is **characterized in that** the composition contains the aforementioned aqueous dispersion containing a polysaccharide particulate gel, the content of the particulate gel is 0.0001 to 1% by weight, and the average particle diameter of the particulate gel is 0.1 to 100 µm.

The ophthalmic composition containing a polysaccharide particulate gel of the present invention can be used specifically as a constituent of an ophthalmic solution. Furthermore, when the ophthalmic composition containing a polysaccharide particulate gel of the present invention is used for a preservative solution for contact lenses, it can control symptoms of dry eye because a contact lens can be put on an eye with the preservative solution attached to the lens and it can stabilize the tear film on the surface of an eyeball for a long time and can keep the tear film smooth.

A drug suitable for a target disease may be blended to the ophthalmic composition containing a polysaccharide particulate gel of the present invention, and examples of the target disease include dry eye syndrome, glaucoma, cataract, inflammation, or pollinosis. It is presumed that the polysaccharide particulate gel according to the present invention makes a drug stay in the internal part or in the vicinity of the surface thereof and demonstrates an effect of efficient and lasting controlled release in an eye.

Examples of the drug include antibacterial agents such as quinolone-based antimicrobials, cephalosporins, sulfacetamide sodium, and sulfamethoxazole; anti-inflammatory agents such as hydrocortisone, dexamethasone, prednisolone, betamethasone, diclofenac, indomethacin, fluorometholone, planoprofen, di-potassium glycyrrhizinate, ε-aminocaproic acid; antihistamic agents such as chlorpheniramine maleate and diphenhydramine hydrochloride; antiglaucomic agents such as prostaglandin derivatives and carbonate dehydrarase inhibitors; antiallergic agents such as sodium cromoglycate; immunosuppressive agents and antimetabolites such as methotrexate, cyclophosphamide, cyclosporine, 6-mercaptoprine, azathioprine, fluorouracil, and tegafur; and antibiotic/anti-inflammatory agent mixtures such as a combination of neomycin sulfate and dexamethasone sodium phosphate; these may be used in combination.

The added amount of the drugs is preferably 0.001 to 10% by weight.

To the ophthalmic composition containing a polysaccharide particulate gel of the present invention may be blended a tonicity adjusting agent, a buffer, a pH regulator, a solubilizing agent, a stabilizer, or a preservative agent in addition to the aforementioned drugs.

Examples of the tonicity adjusting agent include glycerine, propylene glycol, sodium chloride, potassium chloride, sorbitol, or mannitol.

Examples of the buffer include phosphoric acid, phosphoric acid salts, citric acid, acetic acid, ε-aminocaproic acid, or trometamol.

Examples of the pH regulator include hydrochloric acid, citric acid, phosphoric acid, acetic acid, sodium hydroxide, potassium hydroxide, boric acid, borax, sodium carbonate, or sodium hydrogencarbonate.

Examples of the solubilizing agent, which is added when the drug or the additives such as the tonicity adjusting agent is slightly soluble in water, include polysorbate 80, polyoxyethylene hydrogenated castor oil 60, or macrogol 4000.

Examples of the stabilizer include edetic acid or sodium edetate.

Examples of the preservative include sorbic acid, potassium sorbate, benzalkonium chloride, benzethonium chloride, methylparabene, propylparabene, or chlorobutanol, and these may be used in combination.

The content of the polysaccharide particulate gel in the ophthalmic composition containing a polysaccharide particulate gel of the present invention is preferably 0.0001 to 1% by weight, more preferably 0.005 to 0.5% by weight, and even more preferably 0.01 to 0.1% by weight because if it is less than 0.0001% by weight, a desired effect is not demonstrated sufficiently and if it exceeds 1% by weight, the spread or penetration of the composition on or in the surface of an eyeball deteriorates and, as a result, the comfort in use will deteriorate when the composition is used for ophthalmic applications.

The average particle diameter of the polysaccharide particulate gel contained in the ophthalmic composition of the present invention is preferably 0.1 to 100 µm, more preferably 1 to 30 µm, and even more preferably 1 to 10 µm because if the average particle diameter is 100 µm or more, an uncomfortable feeling with foreign substance will generate at the time of dropping and the storage stability of the ophthalmic composition containing a polysaccharide particulate gel will be affected adversely.

The viscosity of the ophthalmic composition containing a polysaccharide particulate gel of the present invention is preferably 0.1 to 30 mPa·s, more preferably 0.5 to 20 mPa·s, and particularly preferably 1 to 10 mPa·s because if it exceeds 30 mPa·s, the comfort in use will deteriorate.

The molecular weight of the polysaccharide contained in the ophthalmic composition of the present invention is preferably 10000 to 1000000, more preferably 20000 to 300000, and even more preferably 30000 to 100000 because if it exceeds 1000000, it becomes difficult to maintain the ophthalmic composition containing a polysaccharide particulate gel and if it is 10000 or less, a desired effect cannot be demonstrated sufficiently.

The present invention provides an aqueous dispersion containing a polysaccharide particulate gel that is a composition in which a particulate material made of a polysaccharide maintains its particle form even if it is heated, in other words, a polysaccharide particulate gel maintains the form of the polysaccharide particulate gel without being dissolved even if it is heated and the polysaccharide particulate gel is dispersed uniformly in an aqueous medium, and a method for producing the same. Moreover, it provides a humectant containing the above-mentioned aqueous dispersion containing a polysaccharide particulate gel excellent in moisture retentivity, ease in handling, and living body safety, and also provides an ophthalmic composition containing an aqueous dispersion containing a polysaccharide particulate gel which can be heat-sterilized and in which the form of the polysaccharide particulate gel is maintained even after sterilization and the physical properties before the sterilization are maintained.

### EXAMPLES

An aqueous dispersion containing a polysaccharide particulate gel, and so on are illustrated specifically below by experimental examples.

### (Example 1)

### (Preparation of an aqueous dispersion containing an agar particulate gel)

Into a hermetic container was weighed 0.5% by weight of agar (AX-30; produced by Ina Food Industry Co., Ltd.). Then, 99.5% by weight of distilled water (distilled water for injection; produced by Otsuka Pharmaceutical Co., Ltd.) was added and the agar was dispersed. Then, the hermetic container was soaked in an oil bath set at 100°C and was stirred at a rotation rate of 700 rpm for 30 minutes by using a mechanical stirrer (BL-600; manufactured by HEIDON) so that the agar was dissolved. Then, the sealed container was taken out from the oil bath and was allowed to cool at room temperature for 210 minutes under stirring at a rotation rate of 700 rpm by using the mechanical stirrer so that an aqueous dispersion containing a particulate gel of agar (henceforth, aqueous dispersion A) was prepared.

### (Irradiation of an aqueous dispersion containing an agar particulate gel with radiation)

The aqueous dispersion A containing particulate agar was collected in an amount of 10 ml, was put into a hermetic container, and was irradiated with γ rays at a dose of 25 kGy so that an aqueous dispersion B was obtained.

### (Test for heat resistance evaluation)

The aqueous dispersion B in which the agar particulate gel had been dispersed uniformly was taken in an amount of 5 ml into a plastic tube and was observed and photographed by an optical microscope (OPTIPHOTO-2; manufactured by Nikon Corp.). Subsequently, the plastic tube was put into an aluminum block thermostatic oven (TAH-1G; manufactured by TAITEC Corp.) set at 95°C and was heated for 30 minutes. Then, the aqueous dispersion B after the heating was observed and photographed by optical microscope.

### (Example 2)

The aqueous dispersion A containing particulate agar was collected in an amount of 10 ml, was put into a hermetic container so that the liquid depth might become 5 mm, and was irradiated with electron rays at an irradiation voltage of 5 MeV and a dose of 25 kGy so that an aqueous dispersion C was obtained. After that, the same test for heat resistance evaluation test as that of Example 1 was carried out.

### (Example 3)

The aqueous dispersion A containing an agar particulate gel was collected in an amount of 10 ml into a hermetic container, was put into a hermetic container so that the liquid depth might become 5 mm, and was irradiated with electron rays at an irradiation voltage of 5 MeV and a dose of 100 kGy so that an aqueous dispersion D was obtained. After that, the same test for heat resistance evaluation test as that of Example 1 was carried out.

### (Example 4)

The same operations as those of Example 1 were carried out except for performing the following autoclaved sterilization test.

### (Autoclaved sterilization test)

The aqueous dispersion B in which the agar particulate gel had been dispersed uniformly was taken into an Erlenmeyer flask made of glass and it was observed and photographed by an optical microscope (OPTIPHOTO-2; manufactured by Nikon Corp.) before heating. Subsequently, the Erlenmeyer flask covered with aluminum foil was put in an autoclave (LSX-500; manufactured by TOMY SEIKO Co., Ltd.) set at 121°C and was heated for 20 minutes. The aqueous dispersion B after the heating was observed and photographed by the optical microscope (OPTIPHOTO-2; manufactured by Nikon Corp.).

### (Comparative Example 1)

### (Preparation of an aqueous dispersion containing an agar particulate gel)

An aqueous dispersion A was prepared by the same method as that of Example 1.

### (Test for heat resistance evaluation)

The aqueous dispersion A in which the agar particulate gel had been dispersed uniformly was taken in an amount of 5 ml into a plastic tube and was observed and photographed by an optical microscope (OPTIPHOTO-2; manufactured by Nikon Corp.). Subsequently, the plastic tube was put into an aluminum block thermostatic oven (TAH-1G; manufactured by TAITEC Corp.) set at 95°C and was heated for 30 minutes. Then, the aqueous dispersion A after the heating was observed and photographed by optical microscope (OPTIPHOTO-2; manufactured by Nikon Corp.).

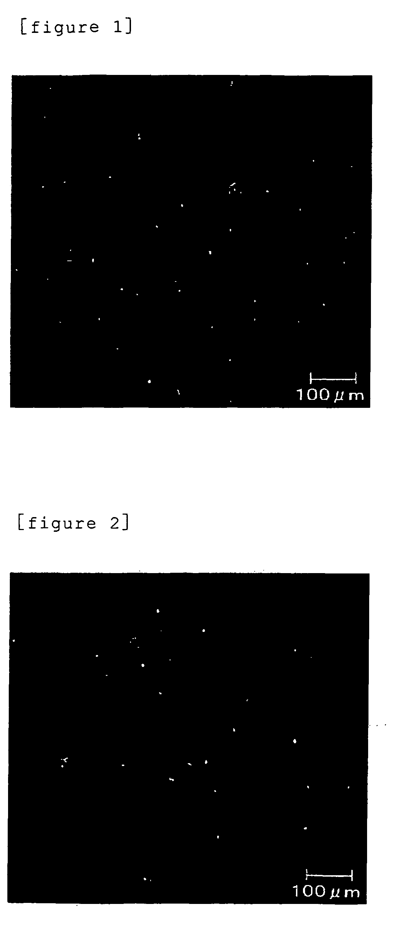

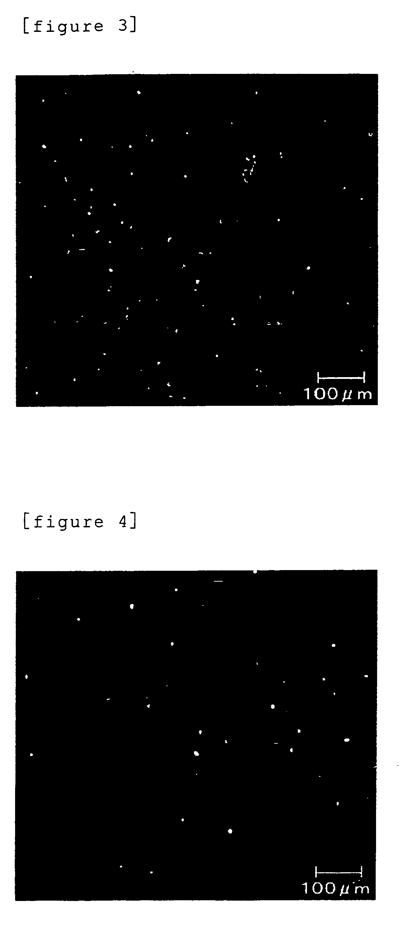

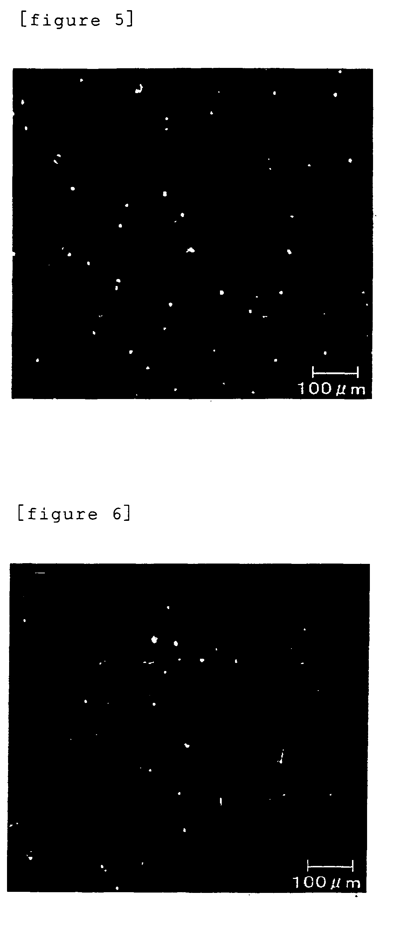

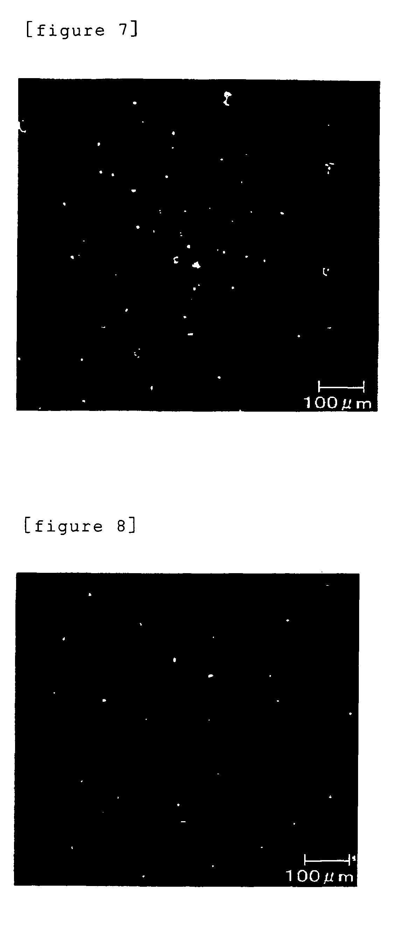

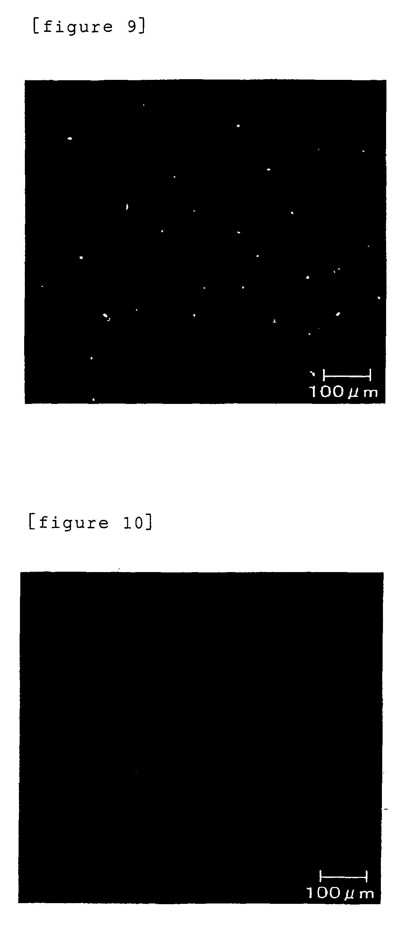

The images of the aqueous dispersion A to D before and after heating in Example 1 to 4 and Comparative Example 1 are shown in Figs. 1 to 10. Each image is processed with image analyzing software and only an agar particular gel is shown. White points in each image are agar particulate gel. In comparing the images of Examples 1 to 4 and the image of Comparative Example 1, it is clear that an agar particulate gel after heating does not dissolve and the form before heating is maintained in the images of Examples 1 to 4.

### (Example 5)

### (Preparation of a γ ray-irradiated agar particulate gel-containing humectant)

An agar powder B was obtained by freeze drying the aqueous dispersion B. Next, an agar solution B was obtained by mixing 1.0% by weight of the agar powder B, 10.0% by weight of glycerine, and 82.9% by weight of distilled water, and dissolving them by heating. A solution prepared by dissolving 0.1% by weight of methyl parahydroxybenzoate in 7.0% by weight of ethanol was added to the agar solution B held at 70°C and then the resultant was allowed to cool to room temperature so that a humectant B was obtained.

### (Comparative Example 2)

### (Preparation of a γ ray-unirradiated agar particulate gel-containing humectant)

An agar powder A was obtained by freeze drying the aqueous dispersion A. Next, an agar solution A was obtained by mixing 1.0% by weight of the agar powder A, 10.0% by weight of glycerine, and 82.9% by weight of distilled water, and dissolving them by heating. A solution prepared by dissolving 0.1% by weight of methyl parahydroxybenzoate in 7.0% by weight of ethanol was added to the agar solution A held at 70°C and then the resultant was allowed to cool to room temperature so that a humectant A was obtained.

### (Comparative Example 3)

### (Preparation of an agar particulate gel-noncontaining humectant)

A solution C was obtained by mixing 11.0% by weight of glycerine and 82.9% by weight of distilled water. To the solution C was added a solution prepared by dissolving 0.1% by weight of methyl parahydroxybenzoate in 7.0% by weight of ethanol so that a humectant C was obtained.

### "Evaluation (1): Smoothness of skin"

Ten expert panelists performed a practical use test of the smoothness of the skin during use and after use by applying humectants A to C to the back of a hand of each panelist. That is, the number of panelists who recognized that the skin during the application and after the application was smooth among the ten expert panelists was examined and the smoothness of the skin was evaluated on a four-graded scale. The evaluation was preformed according to the following criteria:
A: Eight or more expert panelists recognized that the skin during and after use was smooth.
B: Six or more and less than eight expert panelists recognized that the skin during and after use was smooth.
C: Three or more and less than six expert panelists recognized that the skin during and after use was smooth.
D: Less than three expert panelists recognized that the skin during and after use was smooth.

### "Evaluation (2): Non-stickiness of skin"

Ten expert panelists performed a practical use test of the non-stickiness of the skin during use and after use by applying humectants A to C to the back of a hand of each panelist. That is, the number of panelists who recognized that the skin during the application and after the application was non-sticky among the ten expert panelists was examined and the non-stickiness of skin was evaluated on a four-graded scale. The evaluation was preformed according to the following criteria:
A: Eight or more expert panelists recognized that the skin during and after use was non-sticky.
B: Six or more and less than eight expert panelists recognized that the skin during and after use was non-sticky.
C: Three or more and less than six expert panelists recognized that the skin during and after use was non-sticky.
D: Less than three expert panelists recognized that the skin during and after use was non-sticky.

### "Evaluation (3): Measurement of moisture retention effect"

For each of five healthy female subjects, an upper arm inside part was cleaned with ethanol and then 0.5 g of the humectant B was applied to an area with a radius of 3 cm. The conductance of the humectant-applied area at a lapse of 60 minutes since the application was measured with a surface resistance meter (SLT-YKH4101; manufactured by SILTEC) in a room which was conditioned at a temperature of 25°C and a relative humidity of 40%. At this time, it was confirmed that the conductance values of the upper arm inside part skins of the respective subjects before the application of the humectant B were the same value. An average value of the five subjects was taken as a measurement, and evaluation was performed on a four-graded scale on the basis of a conductance measurement obtained. The evaluation criteria are given below.
A: There is a remarkable moisture retention effect. (1800 µs or more)
B: There is a moisture retention effect. (1400 µs or more and less than 1800 µs)
C: A little moisture retention effect is observed. (1000 µs or more and less than 1400 µs)
D: No moisture retention effect is observed. (less than 1000 µs)

The same evaluation of moisture retention effect as that described above was carried out for the humectants A and C.

The results of "Evaluation (1): Smoothness of skin", "Evaluation (2): Non-stickiness of skin", and "Evaluation (3): Measurement of moisture retention effect" are provided in Table 1. It was confirmed from the results provided in Table 1 that the humectant B containing a polysaccharide particulate gel of the present invention had superior properties with respect to the smoothness of the skin exhibited when being applied to the skin and the moisture retention effect in comparison to the humectant C of Comparative Example 3. Moreover, it was revealed from the comparison to Comparative Example 2 that the humectant B containing a polysaccharide particulate gel according to the present invention failed to exhibit stickiness and therefore was excellent in ease in handling and exhibited a high moisture retention effect.

**[Table 1]**

| | Example 5 | Comparative Example 2 | Comparative Example 3 |
|---|---|---|---|
| Evaluation (1) | A | C | B |
| Evaluation (2) | A | D | A |
| Evaluation (3) A value in parentheses is a conductance. | A (1936 µs) | A (2036 µs) | D (898 µs) |

### (Example 6)

### (Preparation of a γ ray-irradiated agar particulate gel-containing ophthalmic solution)

An agar powder B was obtained by freeze drying the aqueous dispersion B. Next, an aqueous agar solution B was obtained by mixing 0.01% by weight of the agar powder B and 97.4% by weight of sterilized purified water, and dissolving them by heating at 40°C. To the aqueous agar solution B kept at 40°C were added 0.01% by weight of pilocarpine hydrochloride and 2.6% by weight of glycerine, followed by stirring for 5 minutes. Then, the pH was adjusted to 7.0 by adding 0.1N sodium hydroxide or 0.1N dilute hydrochloric acid, followed by cooling to room temperature. Thus, an ophthalmic solution B was obtained.

### (Comparative Example 4)

### (Preparation of a γ ray-unirradiated agar particulate gel-containing ophthalmic solution)

An agar powder A was obtained by freeze drying the aqueous dispersion A. Next, an aqueous agar solution A was obtained by mixing 0.01% by weight of the agar powder A and 97.4% by weight of sterilized purified water, and dissolving them by heating at 40°C. To the aqueous agar solution A kept at 40°C were added 0.01% by weight of pilocarpine hydrochloride and 2.6% by weight of glycerine, followed by stirring for 5 minutes. Then, the pH was adjusted to 7.0 by adding 0.1N sodium hydroxide or 0.1N dilute hydrochloric acid, followed by cooling to room temperature. Thus, an ophthalmic solution A was obtained.

### "Evaluation (4): Measurement of viscosity"

First, the ophthalmic solutions A and B prepared, respectively, in Example 6 and Comparative Example 4 were taken in an appropriate amount into glass bottles and their viscosity was measured under conditions of 20°C and 60 rpm by using a B type viscometer (Tokyo Keiki Inc.). A rotor No. 2 was used. Next, the ophthalmic solutions A and B were, respectively, put into glass Erlenmeyer flasks, which were the covered with aluminum foils, put into an autoclave (LSX-500; manufactured by TOMY SEIKO Co., Ltd.) and heated at 121°C for 20 minutes. Then, after cooling to room temperature, the flasks were taken out from the autoclave. Subsequently, appropriate amounts of samples were taken out from the flasks into glass bottles, respectively, and the viscosity of the ophthalmic solutions A and B after heating was measured under conditions of 20°C and 60 rpm by using a B type viscometer (manufactured by Tokyo Keiki Inc.). A rotor No. 2 was used.

The above operation was carried out four times for the ophthalmic solutions A and B, and an average value and a standard deviation of the viscosities before and after the autoclaved sterilization were calculated. The measurements of viscosity in Example 6 and Comparative Example 4 are provided in Table 2. The results provided in Table 2 reveal that the ophthalmic solution B of Example 6 maintains the physical property, i.e., viscosity, of before the autoclaved sterilization also after the sterilization better than the ophthalmic solution A of Comparative Example 4.

**[Table 2]**

| Evaluation (4) | | Before autoclaved sterilization | After autoclaved sterilization |
|---|---|---|---|
| Example 6 | Viscosity average | 1.1 mPa·s | 1.0 mPa·s |
| | Standard deviation | 0.22 | 0.28 |
| Comparative Example 4 | Viscosity average | 1.2 mPa·s | 26.8 mPa·s |
| | Standard deviation | 0.31 | 1.24 |

### INDUSTRIAL APPLICABILITY

The present invention can be used as compositions in the fields of drugs, medical materials, foods, cosmetics, and so on, for example, humectants or ophthalmic solutions.

## Claims

1. An aqueous dispersion containing a polysaccharide particulate gel, wherein the shape of the particulate gel is maintained after the particulate gel has been heated for 30 minutes in an aqueous medium of 95°C.

2. The aqueous dispersion containing a polysaccharide particulate gel according to claim 1, wherein the particulate gel is one that has been irradiated with ionizing radiation in an aqueous medium.

3. The aqueous dispersion containing a polysaccharide particulate gel according to claim 1 or 2, wherein the particulate gel is 500 µm or smaller in particle diameter.

4. The aqueous dispersion containing a polysaccharide particulate gel according to claim 2 or 3, wherein the ionizing radiation is γ rays or electron rays.

5. A method for producing the aqueous dispersion containing a polysaccharide particulate gel according to any one of claims 1 to 4, the method comprising:
a dissolving step of heating a polysaccharide in an aqueous medium to or above a gel transition temperature, thereby dissolving the polysaccharide in the aqueous medium,
a cooling step of cooling the polysaccharide dissolved in the aqueous medium to or below the gel transition temperature while applying an external force, thereby making the polysaccharide into a particulate gel, and
an irradiation step of irradiating the particulate gel in the aqueous medium with ionizing radiation, thereby obtaining an aqueous dispersion containing a polysaccharide particulate gel.

6. The production method according to claim 5, wherein the ionizing radiation is γ rays or electron rays.

7. A humectant containing a polysaccharide particulate gel, wherein the humectant comprises the aqueous dispersion containing a polysaccharide particulate gel according to any one of claims 1 to 5, the content of the particulate gel is 0.01 to 5% by weight, and the average particle diameter of the particulate gel is 1 to 200 µm.

8. The humectant containing a polysaccharide particulate gel according to claim 7, wherein glycerine and/or an alkyl parahydroxybenzoate having an alkyl group having one or two carbon atoms has been added thereto.

9. An ophthalmic composition containing a polysaccharide particulate gel, wherein the composition comprises the aqueous dispersion containing a polysaccharide particulate gel according to any one of claims 1 to 5, the content of the particulate gel is 0.0001 to 1% by weight, and the average particle diameter of the particulate gel is 0.1 to 100 µm.

10. An ophthalmic solution comprising the ophthalmic composition containing a polysaccharide particulate gel according to claim 9.
